(19) **Europäisches Patentamt / European Patent Office / Office européen des brevets**

(11) **EP 4 696 231 A1**

(12) # EUROPÄISCHE PATENTANMELDUNG

(43) Veröffentlichungstag:
**18.02.2026 Patentblatt 2026/08**

(21) Anmeldenummer: **24195005.4**

(22) Anmeldetag: **16.08.2024**

(51) Internationale Patentklassifikation (IPC):
**A61B 5/11** (2006.01)    **A61B 5/00** (2006.01)
**G16H 50/00** (2018.01)

(52) Gemeinsame Patentklassifikation (CPC):
**A61B 5/7267; A61B 5/11; A61B 5/7275;
G16H 40/63; G16H 50/20; G16H 50/70**

(84) Benannte Vertragsstaaten:
**AL AT BE BG CH CY CZ DE DK EE ES FI FR GB
GR HR HU IE IS IT LI LT LU LV MC ME MK MT NL
NO PL PT RO RS SE SI SK SM TR**
Benannte Erstreckungsstaaten:
**BA**
Benannte Validierungsstaaten:
**GE KH MA MD TN**

(71) Anmelder: **Robert Bosch GmbH
70442 Stuttgart (DE)**

(72) Erfinder:
• **WIELAND, Christoph
69168 Wiesloch (DE)**
• **PANKRATIUS, Victor
71638 Ludwigsburg (DE)**

(54) **VORRICHTUNG UND COMPUTERIMPLEMENTIERTES VERFAHREN ZUR VERARBEITUNG VON MIT INSBESONDERE AM KÖRPER GETRAGENEN SENSORELEMENTEN ERFASSTEN SENSORDATEN**

(57)    Vorrichtung und computerimplementiertes Verfahren zur Verarbeitung von mit insbesondere am Körper eines Menschen oder Tieres getragenen Sensorelementen (102) erfassten Sensordaten, insbesondere mit einem Beschleunigungssensor, einem Drehratensensor, einem Geschwindigkeitssensor, einem Magnetometer, einem Drucksensor, einem Blutdrucksensor, einem VO2 Sensor, einem Pulssensor erfasste Sensordaten, wobei je Sensorelement (102) eine Zeitreihe der Sensordaten des Sensorelements (102) bereitgestellt wird, wobei je Zeitreihe ein der Zeitreihe zugeordnetes Merkmal abhängig von den Zeitreihen bestimmt wird, wobei je Zeitreihe mehrere der Zeitreihe zugeordnete Merkmale abhängig von je einem Teil der Zeitreihe bestimmt werden, wobei je Zeitreihe abhängig von den der Zeitreihe zugeordneten Merkmalen ein der Zeitreihe zugeordneter Wert bestimmt wird, wobei abhängig von den den Zeitreihen zugeordneten Werten je Zeitreihe ein der Zeitreihe zugeordneter Zeitreihenwert bestimmt wird, wobei je Zeitreihe abhängig von einem Teil der den Zeitreihen zugeordneten Werte ein der Zeitreihe zugeordneter Zeitreihenwert bestimmt wird, und wobei für wenigstens eine Zeitreihe wenigstens ein der wenigstens einen Zeitreihe zugeordneter Vorhersagewert eines Wertes der wenigstens eine Zeitreihe abhängig von den der wenigstens einen Zeitreihe zugeordneten Zeitreihenwerten bestimmt wird.

Fig. 4

EP 4 696 231 A1

**Beschreibung**

Stand der Technik

Die Erfindung betrifft eine Vorrichtung und ein computerimplementiertes Verfahren zur Verarbeitung von mit insbesondere am Körper getragenen Sensorelementen erfassten Sensordaten.

Die Verarbeitung von Sensordaten ist für eine Vielzahl von nachgelagerten Aufgaben nützlich.

Offenbarung der Erfindung

Ein computerimplementiertes Verfahren zur Verarbeitung von mit insbesondere am Körper eines Menschen oder Tieres getragenen Sensorelementen erfassten Sensordaten, insbesondere mit einem Beschleunigungssensor, einem Drehratensensor, einem Geschwindigkeitssensor, einem Magnetometer, einem Drucksensor, einem Blutdrucksensor, einem VO2 Sensor, einem Pulssensor erfasste Sensordaten, sieht vor, dass je Sensorelement eine Zeitreihe der Sensordaten des Sensorelements bereitgestellt wird, wobei je Zeitreihe ein der Zeitreihe zugeordnetes Merkmal abhängig von den Zeitreihen bestimmt wird, wobei je Zeitreihe mehrere der Zeitreihe zugeordnete Merkmale abhängig von je einem Teil der Zeitreihe bestimmt werden, wobei je Zeitreihe abhängig von den der Zeitreihe zugeordneten Merkmalen ein der Zeitreihe zugeordneter Wert bestimmt wird, wobei abhängig von den den Zeitreihen zugeordneten Werten je Zeitreihe ein der Zeitreihe zugeordneter Zeitreihenwert bestimmt wird, wobei je Zeitreihe abhängig von einem Teil der den Zeitreihen zugeordneten Werte ein der Zeitreihe zugeordneter Zeitreihenwert bestimmt wird, und wobei für wenigstens eine Zeitreihe wenigstens ein der wenigstens einen Zeitreihe zugeordneter Vorhersagewert eines Wertes der wenigstens einen Zeitreihe abhängig von den der wenigstens einen Zeitreihe zugeordneten Zeitreihenwerten bestimmt wird. Die in den multivariaten Zeitreihen enthaltenen Sensordaten werden zum Vorhersagewert verarbeitet. Der Vorhersagewert stellt einen aus dem Kontext der in den multivariaten Zeitreihen enthaltenen Sensordaten generierten Wert dar. Dieser eignet sich besonders zur Weiterverarbeitung, z.B. im Zusammenhang mit der Auswertung von Aktivitäten.

Beispielsweise sind mehrere Zeitreihen einem die Sensorelemente zur Erfassung der mehreren Zeitreihen umfassenden Sensor zugeordnet sind, wobei für wenigstens eine Zeitreihe der der Zeitreihe zugeordnete Zeitreihenwert abhängig von dem Teil der Zeitreihen bestimmt wird, die dem Sensor zugeordnet sind. Dies ermöglicht eine feiner granulierte Auswertung der zeitlichen Charakteristik der jeweiligen Zeitreihe.

Beispielsweise wird für wenigstens ein Sensorelement Meta-Information, insbesondere ein Trageort oder eine Spezifikation des Sensorelements oder demografische Information über einen Träger des Sensorelements, bei der Erfassung der Zeitreihe durch das Sensorelement bereitgestellt wird, wobei die Meta-Information auf eine Repräsentation der Meta-Information abgebildet wird, wobei abhängig von der Repräsentation und dem wenigstens einen Vorhersagewert des Wertes der Zeitreihe ein Signal für die Zeitreihe bestimmt wird. Dies ermöglicht eine Fusion mit zusätzlicher Information.

Eine nachgelagerte Verarbeitung sieht z.B. vor, dass das Signal klassifiziert wird, oder dass abhängig vom Signal ein zukünftiger Wert der Zeitreihe vorhergesagt wird, oder dass abhängig vom Signal ein Wert der Zeitreihe rekonstruiert wird.

Beispielsweise werden die bei der Erfassung der Sensordaten für die Zeitreihe bereitgestellten Werte der Sensordaten durch eine Transformation normiert, wobei die Zeitreihe die normierten Werte umfasst, wobei der zukünftige Wert normiert vorhergesagt wird, und wobei der zukünftige Wert durch Rücktransformation des normiert vorhergesagten Werts bestimmt wird, oder wobei der rekonstruierte Wert normiert rekonstruiert wird, und wobei der rekonstruierte Wert durch Rücktransformation des normiert rekonstruierte Werts bestimmt wird. Dadurch werden statistische Unterschiede in den multivariaten Zeitreihen berücksichtigt. Dies reduziert den Einfluss von Sensordrift in den jeweiligen Sensorelementen insbesondere bei mehrfach nacheinander ausgeführten Berechnungen des Vorhersagewerts für unterschiedliche multivariate Zeitreihen mit dem Verfahren.

Zum Erzeugen von Trainingsdaten ist beispielsweise vorgesehen, dass mehrere Sätze von je einer Zeitreihe und Meta-Information je Sensorelement bereitgestellt werden, wobei die Sätze jeweils in unterschiedlichen Zeiträumen erfasste Sensordaten umfassen, und wobei das Signal je Satz bestimmt wird. Die Signale können zum Training insbesondere eines Klassifizierers zur Klassifizierung des Signals und/oder zum Training zur Vorhersage oder Rekonstruktion wenigstens eines Werts aus einer oder mehreren der Zeitreihen verwendet werden.

Für ein Training wird z.B. ein künstliches neuronales Netzwerk bereitgestellt, wobei ein erster Teil des künstlichen neuronalen Netzwerks ausgebildet ist, das Signal abhängig von den Zeitreihen der Sensordaten der Sensorelemente und abhängig von der Meta-Information zu bestimmen, wobei ein zweiter Teil des künstlichen neuronalen Netzwerks ausgebildet ist, das Signal zu verarbeiten, insbesondere zu klassifizieren oder einen Wert einer der Zeitreihen abhängig vom Signal vorherzusagen oder zu rekonstruieren, wobei das künstliche neuronale Netzwerk, insbesondere der erste Teil des künstlichen neuronalen Netzwerks, auf einem ersten Teil der Sätze dazu trainiert wird, das Signal zu verarbeiten, insbesondere zu klassifizieren oder einen Wert einer der Zeitreihen abhängig vom Signal (312) vorherzusagen oder zu rekonstruieren, und wobei der zweite Teil des künstlichen neuronalen Netzwerks anschließend auf einem zweiten Teil der Sätze dazu trainiert wird, das Signal zu verarbeiten, insbesondere zu klassifizieren oder einen Wert einer der Zeitreihen abhängig vom Signal (312) vorherzusagen oder zu rekonstruieren.

Zur Regularisierung kann vorgesehen sein, dass der Vorhersagewert für wenigstens eine Zeitreihe zusätzlich zur Abhängigkeit von den der wenigstens einen Zeitreihe zugeordneten Zeitreihenwerten in Abhängigkeit von dem der Zeitreihe zugeordneten Wert bestimmt wird.

Zur Vorhersage über eine Zeithorizont kann vorgesehen sein, dass je Zeitreihe mehrere, insbesondere aufeinanderfolgende, der Zeitreihe zugeordneter Zeitreihenwerte bestimmt werden, wobei der wenigstens eine Vorhersagewert für die Zeitreihe abhängig von den je Zeitreihe bestimmten mehreren der Zeitreihenwerte bestimmt wird.

Zur Regularisierung kann vorgesehen sein, dass der der Zeitreihe zugeordnete Wert für wenigstens eine Zeitreihe zusätzlich zur Abhängigkeit von den Merkmalen in Abhängigkeit von der Zeitreihe bestimmt wird.

Eine Vorrichtung zur Verarbeitung von mit insbesondere am Körper getragenen Sensorelementen erfassten Sensordaten, insbesondere mit einem Beschleunigungssensor, einem Drehratensensor, einem Geschwindigkeitssensor, einem Magnetometer, einem Drucksensor, einem Blutdrucksensor, einem VO2 Sensor, einem Pulssensor erfasste Sensordaten, umfasst wenigstens einen Prozessor und wenigstens einen Speicher, wobei der wenigstens eine Speicher vom wenigstens einen Prozessor ausführbare Instruktionen umfasst, bei deren Ausführung durch den wenigstens einen Prozessor die Vorrichtung das Verfahren ausführt.

Ein Computerprogramm umfasst von einem Computer ausführbare Instruktionen, bei deren Ausführung durch den Computer das Verfahren abläuft.

Weitere beispielhafte Ausführungsformen sind der folgenden Beschreibung und der Zeichnung entnehmbar. In der Zeichnung zeigt:

Fig. 1 eine schematische Darstellung einer Vorrichtung zur Verarbeitung von mit insbesondere am Körper getragenen Sensorelementen erfassten Sensordaten,
Fig. 2 eine schematische Darstellung eines Blocks zur Verarbeitung der Sensordaten,
Fig. 3 eine den Block umfassende beispielhafte Architektur zur Verarbeitung der Sensordaten,
Fig. 4 eine beispielhafte Verarbeitung der Sensordaten im Block,
Fig. 5 ein Flussdiagramm mit Schritten eines Verfahrens zur Verarbeitung der Sensordaten.

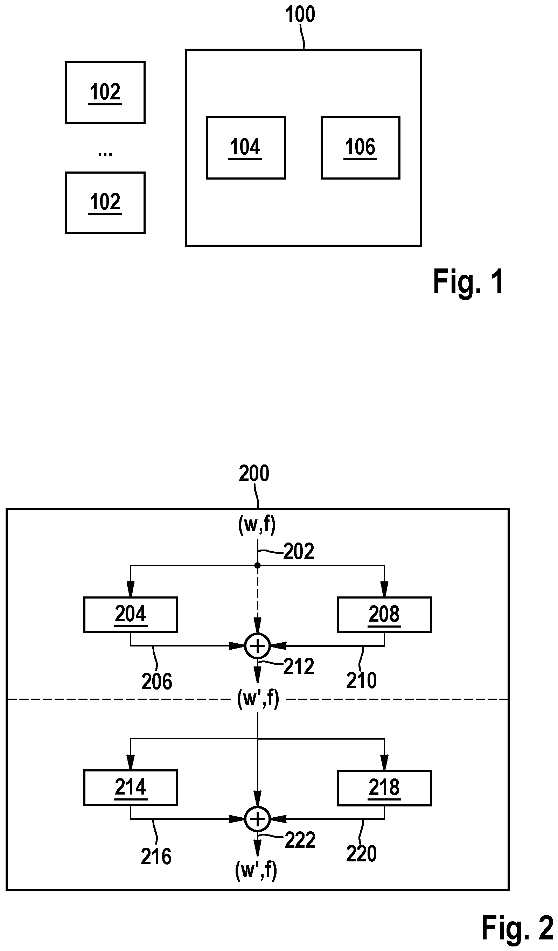

In Figur 1 ist eine Vorrichtung 100 zur Verarbeitung von Sensordaten schematisch dargestellt. Die Sensordaten sind mit insbesondere am Körper getragenen Sensorelementen 102 erfasste Sensordaten.

Beispiele für ein Sensorelement 102 sind ein Beschleunigungssensor, ein Drehratensensor, ein Geschwindigkeitssensor, ein Magnetometer, ein Drucksensor, ein Blutdrucksensor, ein VO2 Sensor, ein Pulssensor.

Es kann vorgesehen sein, dass das Sensorelemente 102 Beschleunigungssensor, oder das Sensorelemente 102 Drehratensensor, oder das Sensorelemente 102 Geschwindigkeitssensor, oder das Sensorelemente 102 Magnetometer, oder das Sensorelemente 102 Drucksensor vom Körper einer Maschine, eines Roboters, eines Fahrzeugs, eines Haushaltsgeräts, eines persönlichen Assistenzsystems oder eines Werkzeugs getragen wird.

Es kann ein Sensor vorgesehen sein, der mehrere Sensorelement 102 umfasst. Beispielweise ist ein dreiachsiger Beschleunigungssensor vorgesehen, der je Achse ein Sensorelement 102 zur Erfassung einer Beschleunigung als Sensorsignal umfasst. Beispielsweise ist ein dreiachsiger Drehratensensor vorgesehen, der je Achse ein Sensorelement 102 zur Erfassung einer Gierrate als Sensordaten umfasst.

Die Vorrichtung 100 umfasst wenigstens einen Prozessor 102 und wenigstens einen Speicher 104.

Der wenigstens eine Speicher 104 umfasst vom wenigstens einen Prozessor 102 ausführbare Instruktionen, bei deren Ausführung durch den wenigstens einen Prozessor 102 die Vorrichtung 100 ein Verfahren zur Verarbeitung von Sensordaten ausführt.

In Figur 2 ist eine schematische Darstellung eines Blocks 200 zur Verarbeitung der Sensordaten dargestellt. Der Block 200 ist ausgebildet, multivariate Zeitreihen 202 zu verarbeiten. Für multivariate Zeitreihen 202, die Sensordaten umfassen, die mit insbesondere am Körper eines Menschen getragenen Sensorelementen 102 erfasst werden, kann die Verarbeitung der multivariaten Zeitreihen 202 mit dem Block 200 dazu verwendet werden, realistische Signale einer Aktivität des Menschen bereitzustellen. Für am Körper eines Tieres getragene Sensorelemente 102 kann der Block 200 entsprechend eingesetzt werden.

Der Block 200 ist ausgebildet f Zeitreihen 202 zu verarbeiten. Die f Zeitreihen 202 weisen jeweils eine Länge w auf. Für f Sensorelemente 102 sind f Zeitreihen 202 vorgesehen, die jeweils von einem der Sensorelemente 102 erfasste Sensordaten umfassen.

Der Block 200 umfasst eine Einrichtung 204 zur Bestimmung von globalen Merkmalen 206, die jeweils einer der Zeitreihen 202 zugeordnet sind.

Die Einrichtung 204 zur Bestimmung der globalen Merkmale 206 ist ausgebildet, die jeweilige Zeitreihe auf ein jeweiliges globales Merkmal, das der jeweiligen Zeitreihe zugeordnet ist, abzubilden.

Die Einrichtung 204 zur Bestimmung der globalen Merkmale 206 ist z.B. ausgebildet je Zeitreihe w globale Merkmale zu bestimmen, die der Zeitreihe zugeordnet sind. Die Einrichtung 204 zur Bestimmung der globalen Merkmale 206 kann ausgebildet sein, je Zeitreihe weniger als w oder mehr als w globale Merkmale zu bestimmen, die der Zeitreihe zugeordnet sind.

Der Block 200 umfasst eine Einrichtung 208 zur Bestimmung von lokalen Merkmalen 210, die jeweils einer der Zeitreihen 202 zugeordnet sind.

Die Einrichtung 208 zur Bestimmung der lokalen Merkmale 210 ist ausgebildet, jeweils einen Teil der jeweilige Zeitreihe auf ein jeweiliges lokales Merkmal, das der jeweiligen Zeitreihe zugeordnet ist, abzubilden.

Die Einrichtung 208 zur Bestimmung der lokalen Merkmale 210 ist z.B. ausgebildet je Zeitreihe w lokale Merkmale zu bestimmen, die der Zeitreihe zugeordnet sind. Die Einrichtung 204 zur Bestimmung der lokalen Merkmale 210 kann ausgebildet sein, je Zeitreihe weniger als w oder mehr als w lokale Merkmale zu bestimmen, die der Zeitreihe zugeordnet sind.

Beispielsweise wird die jeweilige Zeitreihe in die Teile unterteilt. Die Teile sind beispielsweise gleich lang.

Der Block 200 ist ausgebildet, abhängig von den globalen Merkmalen 206 und abhängig von den lokalen Merkmalen 210 insbesondere zukünftige oder rekonstruierte Werte 212 zu bestimmen.

Die Werte 212 umfassen je Zeitreihe wenigstens einen der Zeitreihe zugeordneten Wert. Es kann vorgesehen sein, dass die Werte 212 je Zeitreihe mehrere insbesondere aufeinanderfolgende Werte umfassen, die der jeweiligen Zeitreihe zugeordnet sind.

Der Block 200 ist z.B. für w'=1 ausgebildet, einen einzelnen Wert je Zeitreihe abhängig von dem der jeweiligen Zeitreihe in den globalen Merkmalen 206 zugeordneten Merkmal und abhängig von den der jeweiligen Zeitreihe in den lokalen Merkmalen 210 zugeordneten Merkmalen zu bestimmen. Der Block 200 ist z.B. ausgebildet, einen einzelnen Wert je Zeitreihe abhängig von einer Summe des der jeweiligen Zeitreihe in den globalen Merkmalen 206 zugeordneten Merkmal und der der jeweiligen Zeitreihe in den lokalen Merkmalen 210 zugeordneten Merkmale zu bestimmen.

Beispielsweise ist der Block 200 ausgebildet, für w'>1 je Zeitreihe die einzelnen Werte der mehreren Werte wie für den einzelnen Wert beschrieben zu bestimmen.

Optional ist der Block 200 für den Fall, dass der Block 200 ausgebildet ist, jeweils w'=w globale Merkmale 206 je Zeitreihe und w'=w lokale Merkmale 210 je Zeitreihe zu bestimmen, dazu ausgebildet, die der jeweiligen Zeitreihe zugeordneten Werte zusätzlich zur Abhängigkeit von den globalen Merkmalen 206 und den lokalen Merkmalen 210 abhängig von der jeweiligen Zeitreihe, insbesondere einer Summe der jeweiligen Zeitreihe und der für die jeweilige Zeitreihe bestimmten globalen Merkmale 206 und der für die jeweilige Zeitreihe bestimmten lokalen Merkmale 210 zu bestimmen.

Der Block 200 umfasst eine Einrichtung 214 zur Bestimmung von ersten Zeitreihenwerten 216.

Die Einrichtung 214 zur Bestimmung von ersten Zeitreihenwerten 216 ist ausgebildet, abhängig von den Werten 212 für die f Zeitreihen jeweils w' erste Zeitreihenwerte 216 zu bestimmen.

Die Einrichtung 214 zur Bestimmung von ersten Zeitreihenwerten 216 ist ausgebildet, für w'=1 je Zeitreihe abhängig von den Werten 212 einen einzelnen der ersten Zeitreihenwerte 216 zu bestimmen. Beispielsweise ist die Einrichtung 214 zur Bestimmung von ersten Zeitreihenwerten 216 ausgebildet, für w'>1 je Zeitreihe mehrere der ersten Zeitreihenwerte 216 zu bestimmen, wobei die einzelnen ersten Zeitreihenwerte wie für den einzelnen ersten Zeitreihenwert beschrieben bestimmen werden.

Der Block 200 umfasst eine Einrichtung 218 zur Bestimmung von zweiten Zeitreihenwerten 220.

Die Einrichtung 218 zur Bestimmung von zweiten Zeitreihenwerten 220 ist ausgebildet, jeweils abhängig von einem Teil der Werte 212 für die f Zeitreihen jeweils w' zweite Zeitreihenwerte 220 zu bestimmen.

Die Einrichtung 218 zur Bestimmung von zweiten Zeitreihenwerten 220 ist beispielsweise ausgebildet, für w'=1 je Zeitreihe abhängig von dem Teil der Werte 212 einen einzelnen der zweiten Zeitreihenwerte 220 zu bestimmen. Beispielsweise ist die Einrichtung 218 zur Bestimmung von zweiten Zeitreihenwerten 220 ausgebildet, für w'>1 je Zeitreihe mehrere der zweiten Zeitreihenwerte 220 zu bestimmen, wobei die einzelnen zweiten Zeitreihenwerte wie für den einzelnen zweiten Zeitreihenwert beschrieben bestimmen werden.

Der Block 200 ist ausgebildet, abhängig von den Zeitreihen 202, abhängig von den ersten Zeitreihenwerten 216 und abhängig von den zweiten Zeitreihenwerten 220 wenigstens einen Vorhersagewert 222 zu bestimmen. Der wenigstens eine Vorhersagewert 222 umfasst beispielsweise einen zukünftigen oder einen rekonstruierten Wert der jeweiligen Zeitreihe.

Der Block 200 ist beispielsweise für w'=1 ausgebildet, je Zeitreihe abhängig von der jeweiligen Zeitreihe, abhängig von dem der jeweiligen Zeitreihe in den ersten Zeitreihenwerten 216 zugeordneten einzelnen ersten Zeitreihenwert, und abhängig von dem der jeweiligen Zeitreihe in den zweiten Zeitreihenwerten 220 zugeordneten einzelnen zweiten Zeitreihenwert, einen einzelnen Vorhersagewert für die jeweilige Zeitreihe zu bestimmen. Beispielsweise ist der Block 200 ausgebildet, für w'>1 je Zeitreihe mehrere der Vorhersagewerte 222 zu bestimmen, wobei die einzelnen Vorhersagewerte wie für den einzelnen Vorhersagewert beschrieben bestimmen werden.

Beispielsweise sind die beschriebenen Einrichtungen im Block 200 als Schichten eines künstlichen neuronalen Netzwerks ausgeführt.

Die Schichten des neuronalen Netzwerks sind dazu ausgebildet, die multivariaten Zeitreihen 202 am Eingang des Blocks 200 auf den wenigstens einen Vorhersagewert 222 am Ausgang des Blocks 200 abzubilden.

Die Einrichtungen sind z.B. jeweils als eine einfache Schicht, insbesondere als dense feed forward layer, des künstlichen neuronalen Netzwerks ausgebildet.

Dense feed forward layer ist ein Beispiel für eine Art einer verwendbaren Schicht. Eine andere Art von Schicht kann auch vorgesehen werden. Die Einrichtungen können auch mit mehreren Schichten realisiert sein.

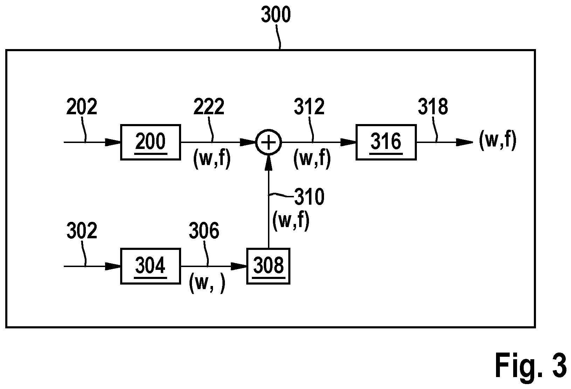

In Figur 3 ist eine den Block 200 umfassende beispielhafte Architektur 300 zur Verarbeitung der Sensordaten dargestellt.

Die Architektur 300 sieht vor, dass mit dem Block 200 abhängig von den Zeitreihen 202 die Vorhersagewerte 222 bestimmt werden. Im Beispiel werden für f Zeitreihen jeweils w Vorhersagewerte bestimmt.

Die Architektur 300 sieht vor, dass ein Trageort 302 der Sensorelemente 102 bei der Erfassung der f Zeitreihen 202 bereitgestellt wird. Der Trageort 302 ist der Name des Orts, z.B. der Name des Körperteils, an dem das jeweilige Sensorelement 102 am Körper angeordnet ist. Das bedeutet, die Sensorelement 102 sind im Beispiel am selben Ort am Körper angeordnet.

Trageort bezieht sich im Beispiel auf den Körper eines Lebewesens, z.B. eines Menschen oder Tiers. Der Trageort beim Menschen ist z.B. Handgelenk, Brust, Hüfte, Knöchel, Taille, Tasche, Kopf.

Die Architektur 300 sieht vor, dass der Trageort 302 mit einem Kodierer 304 auf Repräsentationen 306 abgebildet wird. Im Beispiel werden w identische Repräsentationen 306 für die Zeitreihen 202 der Länge w bestimmt.

Die Architektur 300 sieht vor, dass abhängig von den Repräsentationen 306 mit einem Vervielfältiger 308 eine erweiterte Repräsentation 310 bestimmt wird, die je Vorhersagewert der Vorhersagewerte 222 eine dem jeweiligen Vorhersagewert zugeordnete Repräsentation 306 des Trageorts 302 umfasst.

Die Architektur 300 sieht vor, dass abhängig von den Vorhersagewerten 222 und der erweiterten Repräsentation 310 ein Signal 312 bestimmt wird. Das Signal 312 ist z.B. das realistische Signal der Aktivität des Menschen.

Das Signal 312 wird z.B. abhängig von einer Summe der einzelnen Vorhersagewerte 222 und der einzelnen Werte der erweiterten Repräsentation 310 bestimmt, die einander zugeordnet sind.

Beispielsweise ist die Architektur 300 als Schichten des künstlichen neuronalen Netzwerks ausgeführt. Die Schichten des neuronalen Netzwerks sind dazu ausgebildet, die multivariaten Zeitreihen 202 und die Meta-Information, im Beispiel den Trageort 302, am Eingang der Architektur 300 auf das Signal 312 am Ausgang der Architektur 300 abzubilden.

Die Architektur 300 sieht vor, dass abhängig vom Signal 312 mit einem Kopf 316 eine Ausgangsgröße 318 bestimmt wird. Das bedeutet, die Ausgangsgröße 318 wird ohne die Notwendigkeit weitere aufwändige Datensammlung, abhängig von den multivariaten Zeitreihen 202 bereitgestellt.

Beispielsweise ist der Kopf 316 als Schicht oder als Schichten des künstlichen neuronalen Netzwerks ausgeführt.

Der Kopf 316 ist z.B. ein Klassifizierer, der ausgebildet ist, das Signal 312 zu klassifizieren.

Der Klassifizierer ist z.B. ohne die Notwendigkeit eine weitere aufwändige Datensammlung zu betreiben, abhängig von dem Signal 312 dazu trainierbar, eine Vielfalt von Aktivitäten des Menschen, der die Sensorelemente 102 zur Erfassung der multivariaten Zeitreihen 202 trug, zu erkennen oder nachzuverfolgen.

Die Architektur 300 sieht z.B. vor, dass das Signal 312 mit dem Klassifizierer klassifiziert wird.

Der Kopf 316 ist z.B. wie der Block 200 ausgebildet abhängig vom Signal 312 einen zukünftigen Wert wenigstens einer der Zeitreihen 202 vorherzusagen und/oder abhängig vom Signal 312 einen Wert wenigstens einer der Zeitreihen 202 zu rekonstruieren.

Die Teile der Architektur 300 vor dem Kopf 316 bilden ein Basismodell. Das Basismodell ist ausgebildet, insbesondere abhängig von der Meta-Information, im Beispiel abhängig vom Trageort 302, charakteristische Muster in den multivariaten Zeitreihen 202 zu erkennen. Der Trageort 302 stellt ein Beispiel für Meta-Information über die Platzierung der Sensorelemente 102 dar. Die Meta-Information kann auch eine Spezifikation des jeweiligen Sensorelements 102 oder demografische Information über einen Träger des Sensorelements 102 umfassen. Die Spezifikation ist z.B. eine Abtastrate, eine Empfindlichkeit oder ein Messbereich des jeweiligen Sensorelements 102. Die demografische Information ist z.B. Alter, Geschlecht, Größe, Gewicht des Trägers des Sensorelements 102. Der Kopf 316 ist je nach Aufgabe austauschbar. Es können mehrere verschiedene Köpfe 316 für unterschiedliche Aufgaben zugleich vorgesehen sein. Die mehreren Köpfe 316 sind gleichzeitig miteinander abhängig vom Signal 318 trainerbar.

Die Architektur 300 sieht im Beispiel einen Block 200 vor der Fusion mit der Meta-Information vor. Die Architektur 300 kann auch mehrere Blöcke 200 vor der Fusion vorsehen. Die Architektur 300 sieht beispielsweise einen Block 200 als Kopf 316 nach der Fusion mit der Meta-Information vor. Die Architektur 300 kann auch mehrere Blöcke 200 als Kopf 316 nach der Fusion vorsehen.

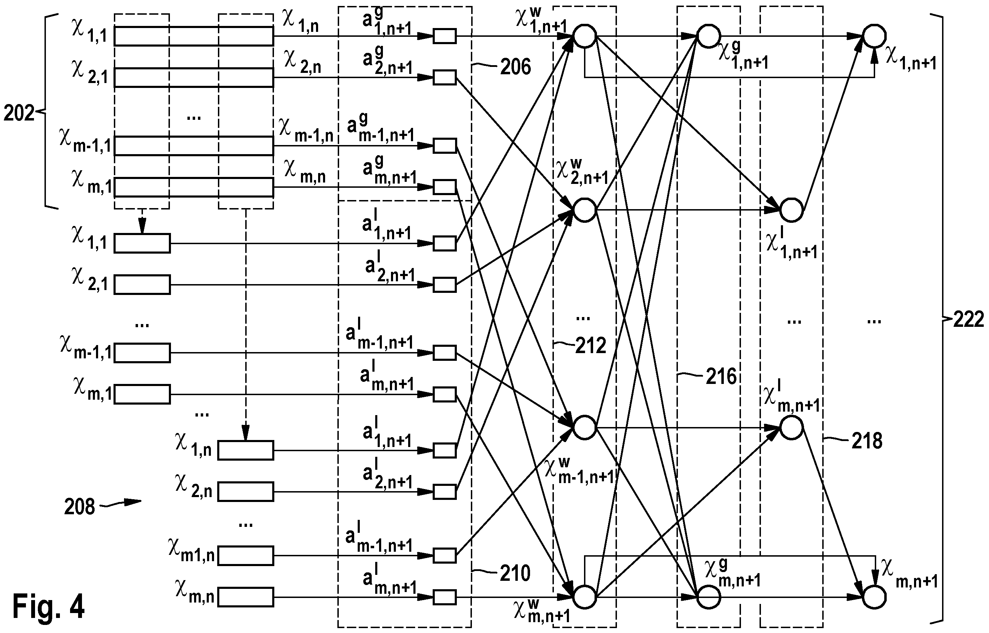

In Figur 4 ist eine beispielhafte Verarbeitung der Sensordaten im Block 200 für m Zeitreihen:

$$x_{1,1}, \dots, x_{1,n}$$

$$x_{2,1}, \dots, x_{2,n}$$

$$x_{m-1,1}, \dots, x_{m-1n}$$

$$x_{m,1}, \dots, x_{m,n}$$

mit der Länge von w=n Werten und je Zeitreihe für einen einzelnen Vorhersagewert $x_{1,n+1}, \dots, x_{m,n+1}$ der Vorhersagewerte 222 schematisch dargestellt.

Die globalen Merkmale 206 sind in Figur 4 mit

$$a_{1,n+1}^{g}, a_{2,n+1}^{g}, \dots, a_{m-1,n+1}^{g}, a_{m,n+1}^{g} \qquad \text{bezeichnet.}$$

Die lokalen Merkmale 210 sind in Figur 4 mit

$$a^l_{1,n+1}, a^l_{2,n+1}, ..., a^l_{m-1,n+1}, a^l_{m,n+1}$$ bezeichnet.

Die für die jeweilige Zeitreihe abhängig von den globalen Merkmalen 206 und abhängig von den lokalen Merkmalen 210 bestimmten Werte 212 sind mit

$$x^w_{1,n+1}, x^w_{2,n+1}, ..., x^w_{m-1,n+1}, x^w_{m,n+1}$$ bezeichnet.

Die ersten Zeitreihenwerte 216 sind in Figur 4 mit $x^g_{1,n+1}, ..., x^g_{m,n+1}$ bezeichnet. Die ersten Zeitreihenwerte 216 werden je Zeitreihe abhängig von den Werten $x^w_{1,n+1}, x^w_{2,n+1}, ..., x^w_{m-1,n+1}, x^w_{m,n+1}$ bestimmt.

Die zweiten Zeitreihenwerte 216 sind in Figur 4 mit $x^l_{1,n+1}, ..., x^l_{m,n+1}$ bezeichnet.

In dem in Figur 4 dargestellten Beispiel sind die ersten beiden Zeitreihen demselben Sensor zugeordnet. Das bedeutet, der zweite Zeitreihenwert der ersten Zeitreihe $x^l_{1,n+1}$ wird abhängig vom der ersten Zeitreihe zugeordneten Wert $x^w_{1,n+1}$ und dem der zweiten Zeitreihe zugeordneten Wert $x^w_{2,n+1}$ bestimmt. Das bedeutet, der zweite Zeitreihenwert der zweiten Zeitreihe $x^l_{2,n+1}$ wird abhängig vom der ersten Zeitreihe zugeordneten Wert $x^w_{1,n+1}$ und dem der zweiten Zeitreihe zugeordneten Wert $x^w_{2,n+1}$ bestimmt. In dem in Figur 4 dargestellten Beispiel sind die letzten beiden Zeitreihen demselben Sensor zugeordnet. Das bedeutet, der zweite Zeitreihenwert der vorletzten Zeitreihe $x^l_{m-1,n+1}$ wird abhängig vom der vorletzten Zeitreihe zugeordneten Wert $x^w_{m-1,n+1}$ und dem der letzten Zeitreihe zugeordneten Wert $x^w_{m,n+1}$ bestimmt. Das bedeutet, der zweite Zeitreihenwert der letzten Zeitreihe $x^l_{m,n+1}$ wird abhängig vom der vorletzten Zeitreihe zugeordneten Wert $x^w_{m-1,n+1}$ und dem der letzten Zeitreihe zugeordneten Wert $x^w_{m,n+1}$ bestimmt.

In einer Anwendung mit dem dreiachsigen Beschleunigungssensor ist beispielsweise vorgesehen, dass die Sensorelemente 102 der drei Achsen, d.h., die drei Zeitreihen, die die Sensordaten aus den drei Sensorelementen 102 des dreiachsigen Beschleunigungssensor umfassen, demselben Sensor zugeordnet sind, und die jeweiligen zweiten Zeitreihenwerte abhängig von den drei Zeitreihenwerten bestimmt werden, die diesen drei Zeitreihen zugeordnet sind.

In einer Anwendung mit dem dreiachsigen Drehratensensor ist beispielsweise vorgesehen, dass die Sensorelemente 102 der drei Achsen, d.h., die drei Zeitreihen, die die Sensordaten aus den drei Sensorelementen 102 des dreiachsigen Drehratensensor umfassen, demselben Sensor zugeordnet sind, und die jeweiligen zweiten Zeitreihenwerte abhängig von den drei Zeitreihenwerten bestimmt werden, die diesen drei Zeitreihen zugeordnet sind.

Die einzelnen Vorhersagewerte $x_{i,n+1}$ werden abhängig von dem für die jeweilige Zeitreihe $i$ bestimmten Wert $x^w_{i,n+1}$, abhängig von dem für die jeweilige Zeitreihe $i$ bestimmten ersten Zeitreihenwert $x^g_{i,n+1}$ und abhängig von dem für die jeweilige Zeitreihe $i$ bestimmten zweiten Zeitreihenwerte $x^l_{i,n+1}$ bestimmt. Dies ist in Figur 4 beispielhaft für den Vorhersagewert $x_{1,n+1}$ für die erste Zeitreihe $i = 1$ und den Vorhersagewert $x_{m,n+1}$ für die letzte Zeitreihe $i = m$ dargestellt.

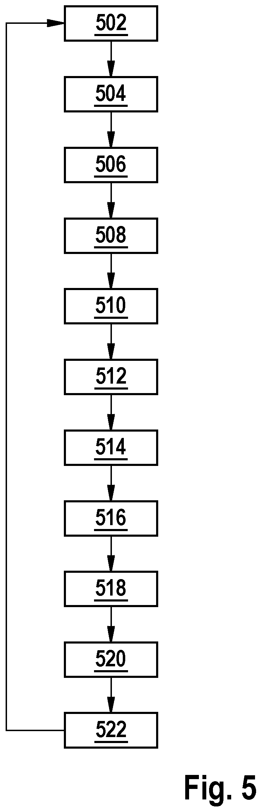

In Figur 5 ist ein Flussdiagramm mit Schritten eines Verfahrens zur Verarbeitung der Sensordaten dargestellt.

Das Verfahren umfasst einen Schritt 502.

Im Schritt 502 wird je Sensorelement 102 eine Zeitreihe der Sensordaten des Sensorelements 102 bereitgestellt. Beispielsweise werden m Zeitreihen mit jeweils n Werten bereitgestellt:

$x_{1,1} ... x_{1,n}; ... ; x_{m,1} ... , x_{m,n}$

Das Verfahren umfasst einen Schritt 504.

Im Schritt 504 wird je Zeitreihe ein der Zeitreihe zugeordnetes Merkmal $a^g_{1,n+1}, ..., a^g_{m,n+1}$ abhängig von den Zeitreihen bestimmt.

Das Verfahren umfasst einen Schritt 506.

Im Schritt 506 werden je Zeitreihe mehrere der Zeitreihe zugeordnete Merkmale $a^l_{1,n+1}, ..., a^l_{m,n+1}$ abhängig von je einem Teil der Zeitreihe bestimmt.

Das Verfahren umfasst einen Schritt 508.

Im Schritt 508 wird je Zeitreihe abhängig von den der Zeitreihe zugeordneten Merkmalen $a^g_{1,n+1}, ..., a^g_{m,n+1}; a^l_{1,n+1}, ..., a^l_{m,n+1}$ ein der Zeitreihe zugeordneter Wert $x^w_{1,n+1}, ..., x^w_{m,n+1}$ bestimmt.

Beispielsweise wird der der Zeitreihe zugeordnete Wert $x^w_{i,n+1}$ für wenigstens eine Zeitreihe $i$ zusätzlich zur Abhängigkeit von den Merkmalen $a^g_{i,n+1}; a^l_{i,n+1}$ in Abhängigkeit von der Zeitreihe $x_{i,1} ... x_{i,n}$ bestimmt.

Das Verfahren umfasst einen Schritt 510.

Im Schritt 510 wird abhängig von den den Zeitreihen zugeordneten Werten $x_{1,n+1}^{w}, ..., x_{m,n+1}^{w}$ je Zeitreihe ein der Zeitreihe zugeordneter Zeitreihenwert $x_{1,n+1}^{g}, ..., x_{m,n+1}^{g}$ bestimmt.

Das Verfahren umfasst einen Schritt 512.

Im Schritt 512 wird je Zeitreihe abhängig von einem Teil der den Zeitreihen zugeordneten Werte $x_{1,n+1}^{w}, ..., x_{m,n+1}^{w}$ ein der Zeitreihe zugeordneter Zeitreihenwert $x_{1,n+1}^{l}, ..., x_{m,n+1}^{l}$ bestimmt.

Beispielsweise sind mehrere Zeitreihen einem die Sensorelemente 102 zur Erfassung der mehreren Zeitreihen umfassenden Sensor zugeordnet. Beispielsweise wird für wenigstens eine Zeitreihe i, die dem Sensor zugeordnet ist, der Zeitreihenwert $x_{i,n+1}^{l}$ abhängig von dem Teil der Zeitreihen bestimmt, die dem Sensor zugeordnet sind.

Das Verfahren umfasst einen Schritt 514.

Im Schritt 514 wird für eine Zeitreihe i ein der wenigstens einen Zeitreihe zugeordneter Vorhersagewert $x_{i,n+1}$ eines Wertes der wenigstens eine Zeitreihe abhängig von den der wenigstens einen Zeitreihe i zugeordneten Zeitreihenwerten $x_{i,n+1}^{g}; x_{i,n+1}^{l}$ bestimmt.

Es kann vorgesehen sein, dass der Vorhersagewert von Werten mehrerer Zeitreihen bestimmt wird.

Es kann vorhergesehen sein, das mehrere Vorhersagewerte je Zeitreihe bestimmt werden.

Beispielsweise werden für die Zeitreihen die den jeweiligen Zeitreihen zugeordnete Vorhersagewerte von Werten der jeweiligen Zeitreihe $x_{1,n+1}. ... , x_{m,n+1}$ bestimmt.

Der Vorhersagewert $x_{i,n+1}$ wird beispielsweise für wenigstens eine Zeitreihe i zusätzlich zur Abhängigkeit von den der wenigstens einen Zeitreihe zugeordneten Zeitreihenwerten $x_{i,n+1}^{g}; x_{i,n+1}^{l}$ in Abhängigkeit von dem der Zeitreihe zugeordneten Wert $x_{i,n+1}^{w}$ bestimmt.

Es kann vorgesehen sein, dass das Verfahren zur Berücksichtigung eines Trageorts einen Schritt 516 umfasst.

Im Schritt 516 wird für wenigstens ein Sensorelement 102 der Trageort 302 des Sensorelements 102 bei der Erfassung der Zeitreihe durch das Sensorelement 102 bereitgestellt.

Es kann vorgesehen sein, dass das Verfahren zur Berücksichtigung eines Trageorts einen Schritt 518 umfasst.

Im Schritt 518 wird der Trageort 302 auf eine Repräsentation 306 des Trageorts 302 abgebildet.

Es kann vorgesehen sein, dass das Verfahren zur Berücksichtigung eines Trageorts einen Schritt 520 umfasst.

Im Schritt 520 wird abhängig von der Repräsentation 306 des Trageorts 302 und dem wenigstens einen Vorhersagewert 222 für die Zeitreihe das Signal 312 für die Zeitreihe bestimmt.

Das Verfahren umfasst einen Schritt 522.

Im Schritt 522 wird z.B. der Vorhersagewert 222, oder zur Berücksichtigung des Trageorts 302, das Signal 312 klassifiziert.

Beispielsweise wird der Kopf 316 abhängig von dem Signal 312 dazu trainiert, die Vielfalt von Aktivitäten des Menschen, der die Sensorelemente 102 zur Erfassung der multivariaten Zeitreihen 202 trug, zu erkennen oder nachzuverfolgen. Beispielsweise wird der Kopf 316 auf den Zeitreihendaten 202 dazu trainiert, die Zeitreihendaten 202 nach einer Aktivität, die bei der Erfassung der Sensordaten vom Träger der Sensorelemente 102 ausgeführt wurde, zu klassifizieren. Beispielsweise wird der Kopf 316 auf den Zeitreihendaten 202 dazu trainiert, die Zeitreihendaten 202 nach einem körperlichen Zustand des Trägers der Sensorelemente 102 bei der Erfassung der Sensordaten zu klassifizieren.

Im Schritt 522 wird z.B. abhängig vom Vorhersagewert 222 der zukünftige Wert der Zeitreihe vorhergesagt oder ein Wert der Zeitreihe rekonstruiert.

Zur Berücksichtigung des Trageorts 302 wird z.B. im Schritt 522 abhängig vom Signal 312 der zukünftige Wert der Zeitreihe vorhergesagt oder ein Wert der Zeitreihe rekonstruiert.

Beispielsweise wird der Kopf 316 abhängig von dem Signal 312 dazu trainiert, einen zukünftigen Wert der Zeitreihe vorherzusagen oder ein Wert der Zeitreihe zu rekonstruieren. Beispielsweise wird der Kopf 316 dazu trainiert, eine Form wenigstens einer der multivariaten Zeitreihen 202 zu rekonstruieren oder vorherzusagen.

Für die Vorhersage wird der Kopf 316 beispielsweise auf den multivariaten Zeitreihen 202 dazu trainiert, n nächste Werte wenigstens einer der multivariaten Zeitreihen 202 vorherzusagen.

Für die Rekonstruktion wird der Kopf 316 beispielsweise auf den multivariaten Zeitreihen 202 dazu trainiert, n maskierte Werte wenigstens einer der multivariaten Zeitreihen 202 vorherzusagen.

Es kann vorgesehen sein, dass der Kopf 316 für den dreiachsigen Beschleunigungssensor oder den dreiachsigen Drehratensensor auf den multivariaten Zeitreihen 202 dazu trainiert wird, abhängig von den Zeitreihen, die die Sensorsignale von zwei der drei Achsen umfassen, die Zeitreihe der Sensordaten der dritten Achse zu bestimmen.

Es kann vorgesehen sein, dass das Signal 312 mit mehreren Köpfen 316 weiterverarbeitet wird. Z.B. wird abhängig vom Signal 312 mit den mehreren Köpfen sowohl das Signal 312 klassifiziert, ein Wert rekonstruiert und/oder ein Wert vorhergesagt. Es kann vorgesehen sein, dass die mehreren Köpfe 316 gleichzeitig miteinander abhängig vom Signal 318 trainiert werden.

Das Training sieht beispielsweise ein Basistraining der Schichten des künstlichen neuronalen Netzwerks abhängig multivariaten Zeitreihen 202 und abhängig von Meta-Information, die z.B. die Position des jeweiligen Sensorelements 102 umfasst, vor.

Das Training sieht beispielsweise ein spezifisches Training des Kopfes 316, d.h., der Ausgabeschicht oder der Ausgabeschichten des neuronalen Netzwerks für eine bestimmte Aufgabe vor. Es kann vorgesehen sein, dass das spezifische Training kann ohne Meta-Information ausgeführt wird.

Das bedeutet, die Bestimmung des Vorhersagewerts 222 und des Signals 312 ist auch ohne Meta-Information möglich.

Es kann vorgesehen sein, dass anschließend der Schritt 502 ausgeführt wird.

Der Schritt 502 kann vorsehen, dass die bei der Erfassung der Sensordaten für die Zeitreihe bereitgestellten Werte der Sensordaten durch eine Transformation normiert werden.

Die Sensordaten werden z.B. mit einem Reverse Instance Normalization layer aus RevIN normiert. Durch das Normieren werden z.B. ein Mittelwert der Sensordaten vom jeweiligen Sensorelement entfernt und eine Varianz in den Werten der Sensordaten vom jeweiligen Sensorelement vereinheitlicht.

Das bedeutet, die Zeitreihen umfassen die normierten Werte.

Beispielsweise wird der zukünftige Wert normiert vorhergesagt und der zukünftige Wert durch Rücktransformation des normiert vorhergesagten Werts bestimmt.

Die Sensordaten werden z.B. mit einem Reverse Instance Denormalization layer aus RevIN normiert. Beispielsweise wird den Sensordaten der jeweilige zuvor entfernte Mittelwert hinzugefügt.

Beispielsweise wird der rekonstruierte Wert normiert rekonstruiert und der rekonstruierte Wert durch Rücktransformation des normiert rekonstruierte Werts bestimmt.

Der Schritt 512 kann vorsehen, dass je Zeitreihe mehrere, insbesondere aufeinanderfolgende, der Zeitreihe zugeordneter Zeitreihenwerte bestimmt werden. Beispielweise wird im Schritt 514 der wenigstens eine Vorhersagewert des Wertes der Zeitreihe abhängig von den je Zeitreihe bestimmten mehreren der Zeitreihenwerte bestimmt.

**Patentansprüche**

1. Computerimplementiertes Verfahren zur Verarbeitung von mit insbesondere am Körper eines Menschen oder Tieres getragenen Sensorelementen (102) erfassten Sensordaten, insbesondere mit einem Beschleunigungssensor, einem Drehratensensor, einem Geschwindigkeitssensor, einem Magnetometer, einem Drucksensor, einem Blutdrucksensor, einem VO2 Sensor, einem Pulssensor erfasste Sensordaten, **dadurch gekennzeichnet, dass** je Sensorelement (102) eine Zeitreihe ($x_{1,1}$ ...

$x_{1,n}, ..., x_{m,1} ... , x_{m,n}$) der Sensordaten des Sensorelements (102) bereitgestellt wird (502), wobei je Zeitreihe ein der Zeitreihe zugeordnetes Merkmal ( $a_{1,n+1}^{g}, ..., a_{m,n+1}^{g}$ ) abhängig von den Zeitreihen bestimmt wird (504), wobei je Zeitreihe mehrere der Zeitreihe zugeordnete Merkmale ( $a_{1,n+1}^{l}, ..., a_{m,n+1}^{l}$ ) abhängig von je einem Teil der Zeitreihe bestimmt werden (506), wobei je Zeitreihe abhängig von den der Zeitreihe zugeordneten Merkmalen ( $a_{1,n+1}^{g}, ..., a_{m,n+1}^{g}; a_{1,n+1}^{l}, ..., a_{m,n+1}^{l}$ ) ein der Zeitreihe zugeordneter Wert ( $x_{1,n+1}^{w}, ..., x_{m,n+1}^{w}$ ) bestimmt wird (508), wobei abhängig von den den Zeitreihen zugeordneten Werten ( $x_{1,n+1}^{w}, ..., x_{m,n+1}^{w}$ ) je Zeitreihe ein der Zeitreihe zugeordneter Zeitreihenwert ( $x_{1,n+1}^{g}, ..., x_{m,n+1}^{g}$ ) bestimmt wird (510), wobei je Zeitreihe abhängig von einem Teil der den Zeitreihen zugeordneten Werte ( $x_{1,n+1}^{w}, ..., x_{m,n+1}^{w}$ ) ein der Zeitreihe zugeordneter Zeitreihenwert ( $x_{1,n+1}^{l}, ..., x_{m,n+1}^{l}$ ) bestimmt wird (512), und wobei für wenigstens eine Zeitreihe wenigstens ein der wenigstens einen Zeitreihe zugeordneter Vorhersagewert ($x_{1,n+1}, ... , x_{m,n+1}$) eines Wertes der wenigstens einen Zeitreihe abhängig von den der wenigstens einen Zeitreihe zugeordneten Zeitreihenwerten ( $x_{1,n+1}^{g}, ..., x_{m,n+1}^{g}; x_{1,n+1}^{l}, ..., x_{m,n+1}^{l}$ ) bestimmt wird (514).

2. Verfahren nach Anspruch 1, **dadurch gekennzeichnet, dass** mehrere Zeitreihen einem die Sensorelemente (102) zur Erfassung der mehreren Zeitreihen umfassenden Sensor zugeordnet sind, wobei für wenigstens eine Zeitreihe der der Zeitreihe zugeordnete Zeitreihenwert ( $x_{1,n+1}^{l}, ..., x_{m,n+1}^{l}$ ) abhängig von dem Teil der Zeitreihen bestimmt wird (512), die dem Sensor zugeordnet sind.

3. Verfahren nach einem der vorherigen Ansprüche, **dadurch gekennzeichnet, dass** für wenigstens ein Sensorelement (102) Meta-Information, insbesondere ein Trageort (302) oder eine Spezifikation des Sensorelements (102) oder demografische Information über einen Träger des Sensorelements (102), bei der Erfassung der Zeitreihe durch das Sensorelement (102) bereitgestellt wird (516), wobei die Meta-Information auf eine Repräsentation (306) der Meta-Information abgebildet wird (518), wobei abhängig von der Repräsentation (306) und dem wenigstens einen Vorhersagewert (222) des Wertes der Zeitreihe ein Signal (312) für die Zeitreihe bestimmt wird (520).

4. Verfahren nach Anspruch 3, **dadurch gekennzeichnet, dass** das Signal (312) klassifiziert (318) wird (522), oder dass abhängig vom Signal (312) ein zukünftiger Wert (318) der Zeitreihe vorhergesagt wird (522), oder dass abhängig vom Signal ein Wert (318) der Zeitreihe rekonstruiert wird (522).

5. Verfahren nach Anspruch 4, **dadurch gekennzeichnet, dass** die bei der Erfassung der Sensordaten für die Zeitreihe bereitgestellten Werte der Sensordaten durch eine Transformation normiert werden (502), wobei die Zeitreihe die normierten Werte umfasst, wobei der zukünftige Wert normiert vorhergesagt und der zukünftige Wert durch Rücktransformation des normiert vorhergesagten Werts bestimmt wird (522), oder wobei der rekonstruierte Wert normiert rekonstruiert und der rekonstruierte Wert durch Rücktransformation des normiert rekonstruierte Werts bestimmt wird (522).

6. Verfahren nach einem der Ansprüche 4 oder 5, **dadurch gekennzeichnet, dass** mehrere Sätze von je einer Zeitreihe und Meta-Information je Sensorelement (102) bereitgestellt werden (502), wobei die Sätze jeweils in unterschiedlichen Zeiträumen erfasste Sensordaten umfassen, und wobei das Signal (312) je Satz bestimmt wird (514).

7. Verfahren nach Anspruch 6, **dadurch gekennzeichnet, dass** ein künstliches neuronales Netzwerk bereitgestellt wird, wobei ein erster Teil (200, 304, 308) des künstlichen neuronalen Netzwerks ausgebildet ist, das Signal (312) abhängig von den Zeitreihen (202) der Sensordaten der Sensorelemente (102) und abhängig von der Meta-Information (302) zu bestimmen, wobei ein zweiter Teil (316) des künstlichen neuronalen Netzwerks ausgebildet ist, das Signal (312) zu verarbeiten (318), insbesondere zu klassifizieren oder einen Wert einer der Zeitreihen abhängig vom Signal (312) vorherzusagen oder zu rekonstruieren, wobei das künstliche neuronale Netzwerk, insbesondere der erste Teil (200, 304, 308) des künstlichen neuronalen Netzwerks, auf einem ersten Teil der Sätze dazu trainiert wird, das Signal (312) zu verarbeiten, insbesondere zu klassifizieren oder einen Wert einer der Zeitreihen abhängig vom Signal (312) vorherzusagen oder zu rekonstruieren, und wobei der zweite Teil (316) des künstlichen neuronalen Netzwerks anschließend auf einem zweiten Teil der Sätze dazu trainiert wird, das Signal (312) zu verarbeiten, insbesondere zu klassifizieren oder einen Wert einer der Zeitreihen abhängig vom Signal (312) vorherzusagen oder zu rekonstruieren.

8. Verfahren nach einem der vorherigen Ansprüche, **dadurch gekennzeichnet, dass** der Vorhersagewert ($x_{i,n+1}$) für wenigstens eine Zeitreihe zusätzlich zur Abhängigkeit von den der wenigstens einen Zeitreihe zugeordneten Zeitreihenwerten ( $x_{i,n+1}^{g}; x_{i,n+1}^{l}$ ) in Abhängigkeit von dem der Zeitreihe zugeordneten Wert ( $x_{i,n+1}^{w}$ ) bestimmt wird (514).

9. Verfahren nach einem der vorherigen Ansprüche, **dadurch gekennzeichnet, dass** je Zeitreihe mehrere, insbesondere aufeinanderfolgende, der Zeitreihe zugeordneter Zeitreihenwerte bestimmt werden (512), wobei der wenigstens eine Vorhersagewert für die Zeitreihe abhängig von den je Zeitreihe bestimmten mehreren der Zeitreihenwerte bestimmt wird (514).

10. Verfahren nach einem der vorherigen Ansprüche, **dadurch gekennzeichnet, dass** der der Zeitreihe zugeordnete Wert ( $x_{i,n+1}^{w}$ ) für wenigstens eine Zeitreihe zusätzlich zur Abhängigkeit von den Merkmalen ( $a_{i,n+1}^{g}; a_{i,n+1}^{l}$ ) in Abhängigkeit von der Zeitreihe ($x_{i,1} \dots x_{i,n}$) bestimmt wird (508).

11. Vorrichtung (100) zur Verarbeitung von mit insbesondere am Körper getragenen Sensorelementen (102) erfassten Sensordaten, insbesondere mit einem Beschleunigungssensor, einem Drehratensensor, einem Geschwindigkeitssensor, einem Magnetometer, einem Drucksensor, einem Blutdrucksensor, einem VO2 Sensor, einem Pulssensor erfasste Sensordaten, **dadurch gekennzeichnet, dass** die Vorrichtung (100) wenigstens einen Prozessor (102) und wenigstens einen Speicher (104) umfasst, wobei der wenigstens eine Speicher (104) vom wenigstens einen Prozessor (102) ausführbare Instruktionen umfasst, bei deren Ausführung durch den wenigstens einen Prozessor (102) die Vorrichtung (100) das Verfahren nach einem der Ansprüche 1 bis 10 ausführt.

12. Computerprogramm, **dadurch gekennzeichnet, dass** das Computerprogramm von einem Computer ausführbare Instruktionen umfasst, bei deren Ausführung durch den Computer das Verfahren nach einem der Ansprüche 1 bis 10 abläuft.

100

102

...

102

104     106

**Fig. 1**

200

(w,f)

202

204     208

206     212     210

(w',f)

214     218

216     222     220

(w',f)

**Fig. 2**

Fig. 3

Fig. 4

Fig. 5

## EUROPÄISCHER RECHERCHENBERICHT

### EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (IPC) |
|---|---|---|---|
| X | US 2022/022807 A1 (MIKHAIL GEORGE [US] ET AL) 27. Januar 2022 (2022-01-27) * das ganze Dokument * ----- | 1-12 | INV. A61B5/11 A61B5/00 G16H50/00 |

RECHERCHIERTE SACHGEBIETE (IPC)

A61B
G16H

Der vorliegende Recherchenbericht wurde für alle Patentansprüche erstellt

| Recherchenort | Abschlußdatum der Recherche | Prüfer |
|---|---|---|
| Berlin | 27. Januar 2025 | Clevorn, Jens |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus anderen Gründen angeführtes Dokument
..........................................................................................
& : Mitglied der gleichen Patentfamilie, übereinstimmendes Dokument

EPO FORM 1503 03.82 (P04C03)

**ANHANG ZUM EUROPÄISCHEN RECHERCHENBERICHT**
**ÜBER DIE EUROPÄISCHE PATENTANMELDUNG NR.**

EP 24 19 5005

In diesem Anhang sind die Mitglieder der Patentfamilien der im obengenannten europäischen Recherchenbericht angeführten Patentdokumente angegeben.
Die Angaben über die Familienmitglieder entsprechen dem Stand der Datei des Europäischen Patentamts am
Diese Angaben dienen nur zur Unterrichtung und erfolgen ohne Gewähr.

27-01-2025

| Im Recherchenbericht angeführtes Patentdokument | Datum der Veröffentlichung | Mitglied(er) der Patentfamilie | Datum der Veröffentlichung |
|---|---|---|---|
| US 2022022807 A1 | 27-01-2022 | AU 2021312298 A1 | 23-03-2023 |
| | | CA 3189564 A1 | 27-01-2022 |
| | | CN 116157058 A | 23-05-2023 |
| | | EP 4185190 A1 | 31-05-2023 |
| | | JP 2023536243 A | 24-08-2023 |
| | | US 2022022807 A1 | 27-01-2022 |
| | | WO 2022018652 A1 | 27-01-2022 |

EPO FORM P0461

Für nähere Einzelheiten zu diesem Anhang : siehe Amtsblatt des Europäischen Patentamts, Nr.12/82